# EUROPEAN PATENT APPLICATION

(11) **EP 4 109 096 A1**
(43) Date of publication of application: **28.12.2022**
(21) Application number: 21756197.6
(22) Date of filing: 08.02.2021
(51) Int. Cl.: G01N 33/53, C12Q 1/06, C12Q 1/6806, G01N 33/543

(54) **METHOD FOR ANALYZING BIOLOGICAL SAMPLE**

(30) Priority: 17.02.2020 JP 2020024511
(71) Applicant: JVCKenwood Corporation, Yokohama-shi, Kanagawa 2210022 (JP)
(72) Inventor: TSUJITA Koji, Yokohama-shi, Kanagawa 221-0022 (JP); ITONAGA Makoto, Yokohama-shi, Kanagawa 221-0022 (JP)
(74) Representative: Carstens, Dirk Wilhelm
(86) International application number: PCT/JP2021/004553
(87) International publication number: WO 2021/166713

(57) **Abstract**

An exosome to be analyzed having a first bead and a second bead bound thereto is collected from a buffer fluid containing the exosome to be analyzed having the first bead and the second bead bound thereto. A first antibody that specifically binds to a first antigen associated with a first disease is fixed to a surface of the first bead. A second antibody that specifically binds to a second antigen associated with a second disease is fixed to a surface of the second bead. The first bead is separated from the exosome, and the exosome having the second bead bound thereto is collected. The exosome having the second bead bound thereto is dissolved, and the second bead and an inclusion of the exosome are collected (S19 and S20). The inclusion of the exosome is analyzed (S21), and the number of second beads is counted (S22).

## Description

### [TECHNICAL FIELD]

The present disclosure relates to a biological sample analysis method of analyzing inclusions of exosomes contained in a biological sample, and counting the number of exosomes.

### [BACKGROUND ART]

Antigens associated with specific diseases are detected and analyzed as biomarkers to detect specific diseases or to verify the efficacy of treatments for specific diseases. Exosomes, which are secreted from cells and contained in various body fluids such as blood, are promising as biomarkers for detecting specific diseases.

Exosomes are minute membrane vesicles covered with a lipid bilayer, which contains various membrane proteins such as CD9 and CD63. It is known that when a person suffers from a specific disease, there is an increase in the number of exosomes in which disease-related membrane proteins are expressed on the surface of the lipid bilayer. By counting the number of exosomes with disease-related antigens using disease-related membrane proteins expressed on the surface of exosomes as antigens, it is possible to detect whether a person has a specific disease or not.

Inside exosomes, there are nucleic acids such as miRNA (microRNA) and DNA, and inclusions such as proteins. Inclusions of exosomes are also promising as biomarkers for the detection of specific diseases (see Patent Literature 1) .

### [CITATION LIST]

### [PATENT LITERATURE]

Patent Literature 1: Japanese Unexamined Patent Application Publication No. 2018-163043
Patent Literature 2: Japanese Unexamined Patent Application Publication No. 2017-40595
Patent Literature 3: Japanese Unexamined Patent Application Publication No. 2019-211366

### [SUMMARY OF INVENTION]

Conventionally, two analytes for a biological sample containing exosomes are prepared, and the inclusions of exosomes are analyzed in one sample, and the number of exosomes are counted in the other sample. In other words, the exosomes in which the inclusions are analyzed are not the same exosomes in which the number thereof is counted. It is desirable to analyze inclusions of exosomes and count the number of exosomes using one analyte.

One or more embodiments aim to provide a biological sample analysis method capable of analyzing inclusions of exosomes and counting the number of exosomes using only one biological sample.

An aspect of one or more embodiments provides a biological sample analysis method including: injecting, into a reaction container, a first buffer fluid containing a plurality of a first bead having fixed to a surface thereof a first antibody that specifically binds to a first antigen associated with a first particular disease; injecting, into the reaction container, a plurality of a second bead having fixed to a surface thereof a second antibody that specifically binds to a second antigen associated with a second particular disease, and a biological sample containing an exosome to be analyzed having the first antigen and the second antigen on a surface of the exosome, thereby generating a second buffer fluid containing the exosome to be analyzed having the first bead and the second bead bound thereto; collecting the exosome to be analyzed having the first bead and the second bead bound thereto from the second buffer fluid; separating the first bead from the collected exosome to be analyzed having the first bead and the second bead bound thereto, and collecting the exosome to be analyzed having the second bead bound thereto; dissolving the collected exosome to be analyzed having the second bead bound thereto, to be separated into the second bead and an inclusion of the exosome to be analyzed, and collecting the second bead and the inclusion of the exosome to be analyzed; and analyzing the inclusion of the collected exosome to be analyzed, and counting the number of collected second beads.

The biological sample analysis method according to one or more embodiments makes it possible to analyze inclusions of exosomes and count the number of exosomes using only one biological sample.

### [BRIEF DESCRIPTION OF DRAWINGS]

FIG. 1A is a flowchart partially illustrating a biological sample analysis method according to one or more embodiments.
FIG. 1B is a flowchart partially illustrating the biological sample analysis method according to one or more embodiments following FIG. 1A.
FIG. 2 is a conceptual diagram illustrating a magnetic microbead for use in the biological sample analysis method according to one or more embodiments.
FIG. 3 is a conceptual view illustrating a reaction container injected with buffer fluid containing magnetic microbeads.
FIG. 4 is a conceptual diagram illustrating an analyte of a biological sample containing single-positive exosomes and double-positive exosomes.
FIG. 5A is a conceptual diagram illustrating the structure of the single-positive exosome.
FIG. 5B is a conceptual diagram illustrating the structure of the double-positive exosome.
FIG. 6 is a conceptual diagram illustrating antibody nanobeads for use in the biological sample analysis method according to one or more embodiments.
FIG. 7 is a conceptual diagram illustrating an analyte containing double-positive exosomes and antibody nanobeads injected into a reaction container injected with buffer fluid containing a magnetic microbead, and the double-positive exosomes sandwiched between the magnetic microbead and the antibody nanobeads.
FIG. 8 is a conceptual diagram of the reaction container illustrated in FIG. 7 after removal of unwanted materials other than the magnetic microbead and the antibody nanobeads which sandwich the double-positive exosomes.
FIG. 9 is a diagram illustrating a method of separating the unwanted materials from the reaction container illustrated in FIG. 7.
FIG. 10 is a conceptual diagram illustrating a state where the exosomes from among the magnetic microbead and the antibody nanobeads which sandwiched the double-positive exosomes are detached from the magnetic microbead.
FIG. 11 is a diagram illustrating a method of detaching the exosomes from the magnetic microbead using a hapten.
FIG. 12 is a diagram illustrating a method of detaching the exosomes from the magnetic microbead using a linker.
FIG. 13 is a conceptual diagram illustrating the magnetic microbead obtained by separating the magnetic microbead, the antibody nanobeads capturing the double-positive exosomes, and the single-positive exosomes.
FIG. 14 is a conceptual diagram illustrating the antibody nanobeads capturing the double-positive exosomes, and the single-positive exosomes, obtained by separating the magnetic microbead, the antibody nanobeads capturing the double-positive exosomes, and the single-positive exosomes.
FIG. 15A is a diagram illustrating the steps up to the middle of the method of separating the magnetic microbeads and the antibody nanobeads.
FIG. 15B is a diagram illustrating the steps following those in FIG. 15A of the method of separating the magnetic microbeads and the antibody nanobeads.
FIG. 16 is a conceptual diagram illustrating the antibody nanobeads capturing the double-positive exosomes, obtained by separating the antibody nanobeads capturing the double-positive exosomes, and the single-positive exosomes.
FIG. 17 is a conceptual diagram illustrating the single-positive exosomes obtained by separating the antibody nanobeads capturing the double-positive exosomes, and the single-positive exosomes.
FIG 18 is a conceptual diagram illustrating the antibody nanobeads detached from the double-positive exosomes.
FIG. 19 is a conceptual diagram illustrating the antibody nanobeads obtained by detaching the antibody nanobeads and the double-positive exosomes.
FIG. 20 is a conceptual diagram illustrating the double-positive exosomes obtained by detaching the antibody nanobeads and the double-positive exosomes.
FIG. 21 is a flowchart illustrating a specific method of counting the number of antibody nanobeads in step S22 of FIG. 1B.
FIG. 22 is a diagram illustrating the method of counting the number of antibody nanobeads.
FIG. 23 is a conceptual diagram illustrating the formation of polar functional groups for attaching the antibody nanobeads to a disk substrate.

### [DESCRIPTION OF EMBODIMENTS]

Hereinafter, a biological sample analysis method according to one or more embodiments will be described with reference to the accompanying drawings. In one or more embodiments, an example will be described regarding a biological sample analysis method capable of capturing double-positive exosomes in which two specific membrane proteins are expressed on the surface of one exosome, thereby analyzing inclusions of the double-positive exosomes, and counting the number of double-positive exosomes.

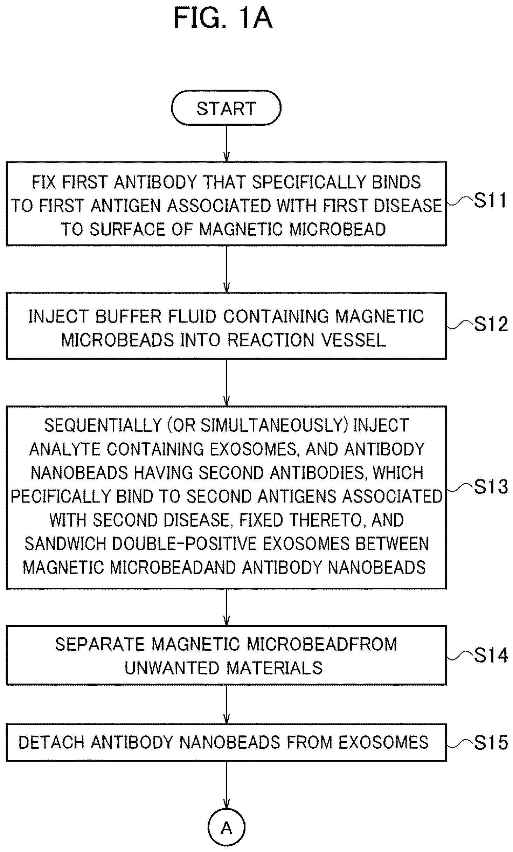

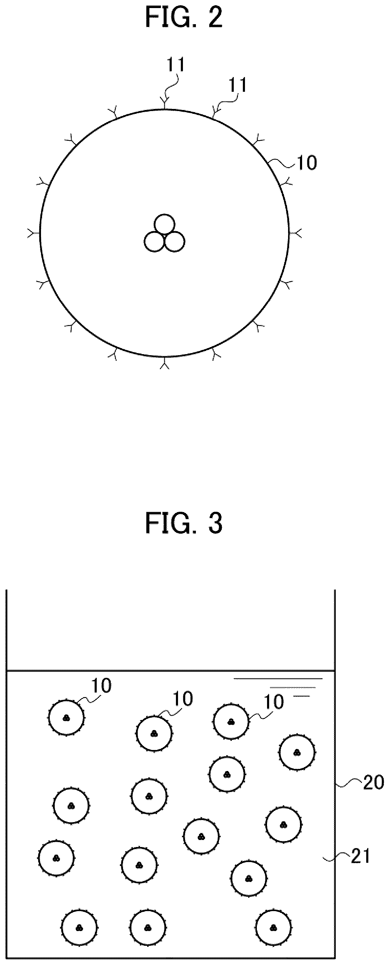

In FIG. 1A, an operator, in step S11, fixes a plurality of antibodies 11 that specifically bind to first disease-associated antigens to the surface of a magnetic microbead 10, as illustrated in FIG. 2. As illustrated in FIG. 3, in step S12, the operator injects buffer fluid 21 (first buffer fluid) containing a number of magnetic microbeads 10 to which the antibodies 11 are fixed into a reaction container 20. The diameters of the magnetic microbeads 10 may be about 3 µm, and the diameters are preferably on the order of micrometers.

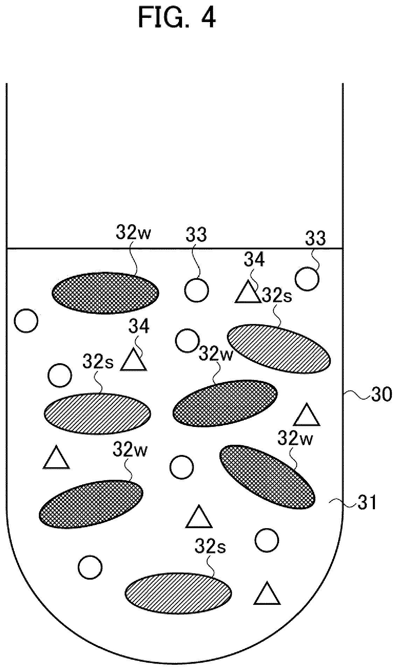

As illustrated in FIG. 4, the operator prepares an analyte 31 of a biological sample in a container 30. The analyte 31 contains single-positive exosome 32s and double-positive exosomes 32w, which will be described below. The analyte 31 is optional fluid such as blood, and contains contaminants 33 and 34 which are unwanted materials in addition to the single-positive exosome 32s and the double-positive exosomes 32w. The single-positive exosomes 32s and the double-positive exosomes 32w are collectively called the exosomes 32.

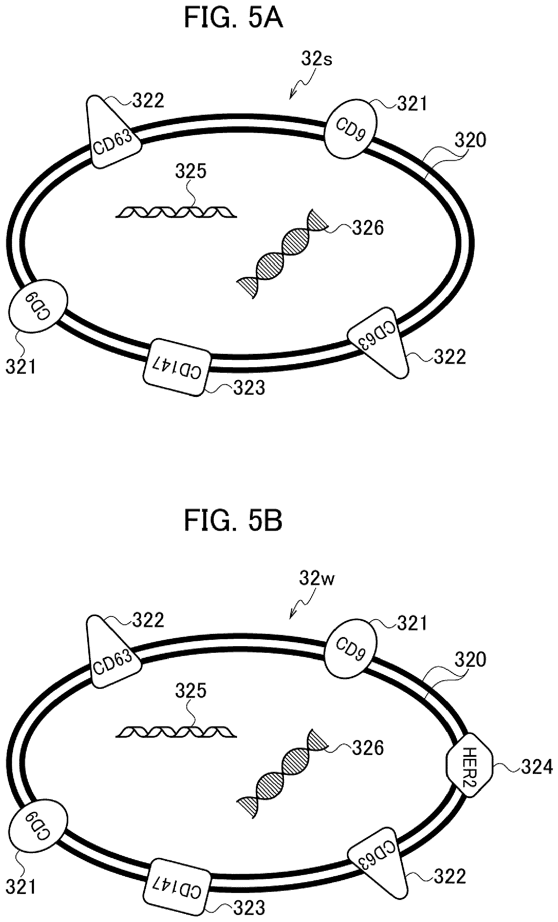

As illustrated conceptually in FIG. 5A, the single-positive exosome 32s is covered with a lipid double membrane 320, and the lipid double membrane 320 contains, for example, membrane proteins 321 to 323. The membrane protein 321 is CD9, the membrane protein 322 is CD63, and the membrane protein 323 is CD147. CD9 and CD63 are membrane proteins present in normal exosomes, and CD147 is an example of a first disease-associated membrane protein. Thus, the single-positive exosome 32s refers to an exosome 32 in which one disease-associated membrane protein (that is, CD147 in one or more embodiments) is present on the surface of one exosome 32.

As illustrated conceptually in FIG. 5B, the double-positive exosome 32w is covered with the lipid double membrane 320, and the lipid double membrane 320 contains, for example, membrane proteins 321 to 324. The membrane protein 324 is HER2 (CD340), which is an example of a second disease-associated membrane protein. Thus, the double-positive exosome 32w refers to an exosome 32 in which two disease-associated membrane proteins (that is, CD147 and HER2 (CD340) in one or more embodiments) are present on the surface of one exosome 32. Hereinafter, HER2 (CD340) is referred to using the common name HER2.

In one or more embodiments, by way of example, the double-positive exosome 32w having both CD 147 and HER2 present on the surface is the exosome to be analyzed. In one or more embodiments, CD 147 is used as a first antigen associated with a first disease and HER2 is used as a second antigen associated with a second disease, but the present invention is not limited thereto. The antibody 11 fixed to the surface of the magnetic microbead 10 is a first antibody that specifically binds to CD147.

Inside the exosome 32 are nucleic acids such as miRNA 325 and DNA 326, and unillustrated inclusions such as proteins.

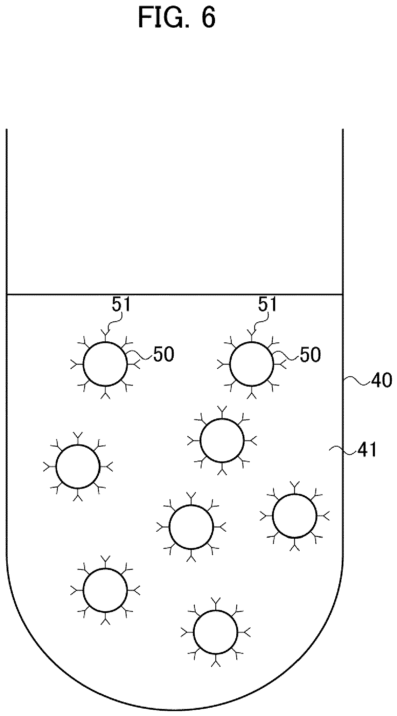

As illustrated in FIG. 6, the operator prepares in the container 40, buffer fluid 41 containing antibody nanobeads 50 having a plurality of antibodies 51 fixed to the surface thereof. The antibody nanobeads 50 are much smaller in diameter than the magnetic microbeads 10 and have a diameter of about 200 nm. The diameter of the antibody nanobeads 50 may be less than 1 µm, preferably from 100 nm to 500 nm. The antibody 51 fixed to the surface of the antibody nanobead 50 is an antibody (second antibody) that specifically binds to HER2, which is the second antigen associated with the second disease.

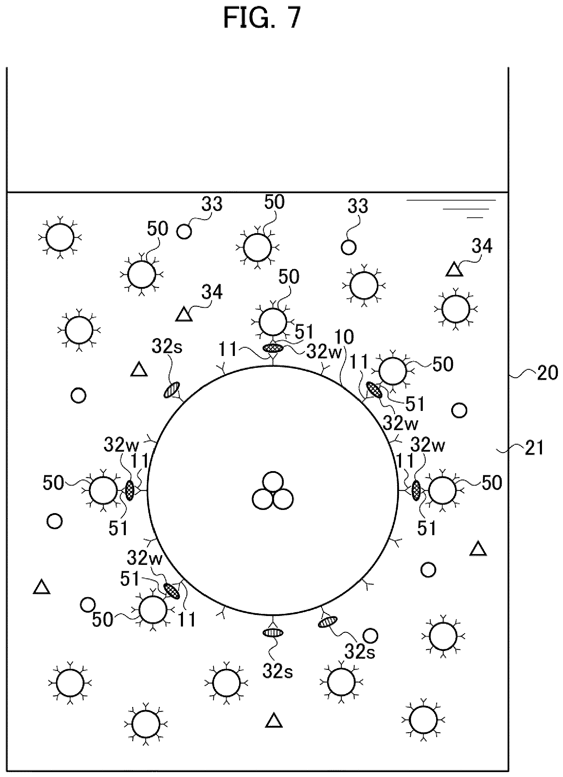

As illustrated in FIG. 7, the operator sequentially (or simultaneously) injects the analyte 31 containing the exosomes 32, and the buffer fluid 41 containing the antibody nanobeads 50 into the reaction container 20 in step S13. When the buffer fluid 21 is a mixture of the buffer fluid 21 and the buffer fluid 41, buffer fluid 21 (second buffer fluid), which contains the double-positive exosomes 32w sandwiched between the magnetic microbead 10 and the antibody nanobeads 50, is generated in the reaction container 20. In this case, since "sandwich" means that the two beads bind to one exosome, it is possible to say that second buffer fluid which contains the exosomes 32w having the magnetic microbead 10 and the antibody nanobeads 50 bound thereto is generated. At this time, the operator shakes the reaction container 20 as necessary and waits for a predetermined time.

In FIG. 7, the double-positive exosomes 32w in which both CD 147 and HER2 are present on the surface are sandwiched between the magnetic microbead 10 and the antibody nanobeads 50. The single-positive exosomes 32s in which HER2 is not present bind to the antibodies 11 of the magnetic microbead 10. However, the single-positive exosome 32s are not sandwiched between the magnetic microbead 10 and the antibody nanobeads 50 because the antibody nanobeads 50 do not bind to the single-positive exosome 32s.

In FIG. 7, there are a number of magnetic microbeads 10 in the reaction container 20 in reality, but for ease of understanding, only an enlarged single magnetic microbead 10 is illustrated.

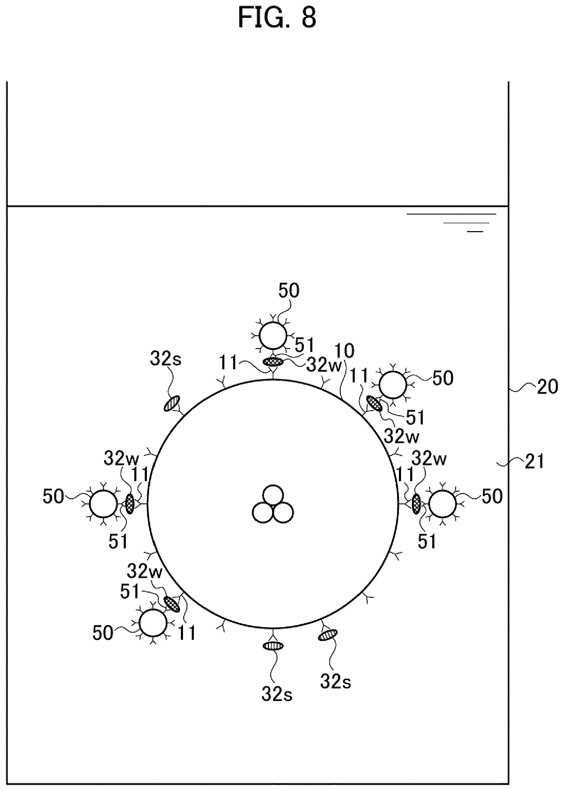

In step S14, the operator separates the magnetic microbead 10 to which the antibody nanobeads 50 are connected via the exosomes 32 from the unwanted materials by means of magnetic collection, weak centrifugation, or the like. The operator washes the separated magnetic microbead 10. The antibody nanobeads 50 that are not connected to the magnetic microbead 10, and the contaminants 33 and 34 are unwanted materials. Since the antibody nanobeads 50 are excessively injected in step S13, the unwanted materials are mainly the antibody nanobeads 50 not connected to the magnetic microbead 10. As a result of step S14, the magnetic microbead 10 to which the antibody nanobeads 50 are connected via the exosomes 32 can be collected, as illustrated in FIG. 8.

The exosomes 32s not having the antibody nanobeads 50 bound thereto are also bound to the collected magnetic microbead 10. Of course, among the plurality of magnetic microbeads 10, there are magnetic microbeads 10 having bound thereto only the exosomes 32w which are bound to the antibody nanobeads 50, and magnetic microbeads 10 having bound thereto only the exosomes 32s which are not bound to the antibody nanobeads 50.

Meanwhile, in a case where the antibody nanobeads 50 are not magnetic nanobeads, the magnetic microbead 10 can be easily separated from the antibody nanobeads 50 which are not connected to the magnetic microbead 10 by magnetically collecting the magnetic microbead 10. When the number of the antibody nanobeads 50 connected to the magnetic microbead 10 is counted in step S22 described later, the antibody nanobeads 50 are preferably magnetic nanobeads. Even if the antibody nanobeads 50 are magnetic nanobeads, it is possible to separate the magnetic microbead 10 from the unconnected antibody nanobeads 50.

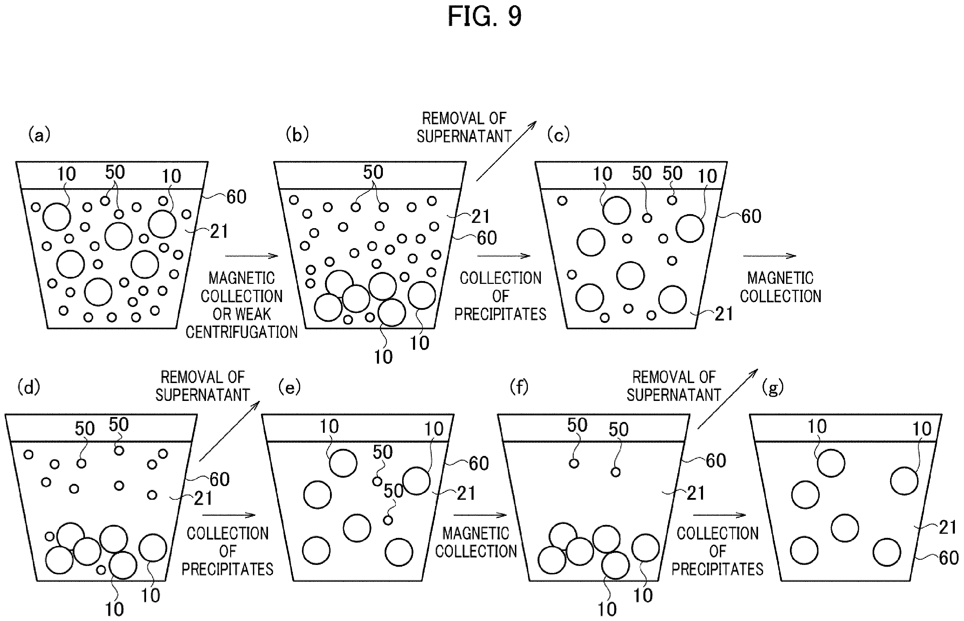

Specifically, as illustrated in FIG. 9, it is possible to separate the magnetic microbeads 10 from the unconnected antibody nanobeads 50. In FIG. 9, (a) illustrates a state of a container 60 injected with the buffer fluid 21 which contains the magnetic microbeads 10 to which the antibody nanobeads 50 are connected and the unconnected antibody nanobeads 50. Part (a) of FIG. 9 corresponds to the state of FIG. 7. Magnetic collection in which a magnet is placed at the bottom of the container 60 or weak centrifugation can mostly separate the magnetic microbeads 10 from the unconnected antibody nanobeads 50, as illustrated in (b) of FIG. 9

This is because the volumes of the magnetic microbeads 10 and the antibody nanobeads 50 differ by about 1000 times, which means that the magnetic microbeads 10 are magnetically collected in a short time, while the antibody nanobeads 50 are magnetically collected only after a long time. By utilizing the time difference in this magnetic collection, the magnetic microbeads 10 and the unconnected antibody nanobeads 50 can be mostly separated.

In the step of washing the magnetic microbeads 10, when the precipitates, which are mainly the magnetic microbeads 10 in (b) of FIG. 9, are collected, the supernatant is removed, and the buffer fluid 21 is added, the number of unconnected antibody nanobeads 50 can be greatly reduced as illustrated in (c) of FIG. 9. When magnetic collection is performed in the state illustrated in (c) of FIG. 9, (d) of FIG. 9 is achieved, and when the precipitates are collected, the supernatant is removed, and the buffer fluid 21 is added, the unconnected antibody nanobeads 50 can be further greatly reduced as illustrated in (e) of FIG. 9.

When magnetic collection is performed in the state illustrated in (e) of FIG. 9, (f) of FIG. 9 is achieved, and when the precipitates are collected, the supernatant is removed, and the buffer fluid 21 is added, it is possible to separate only the magnetic microbeads 10 to which the antibody nanobeads 50 are connected as illustrated in (g) of FIG. 9. Part (g) of FIG. 9 corresponds to the state of FIG. 8. The number of times the precipitates are collected and the supernatant is removed is not limited to the number of times illustrated in FIG. 9.

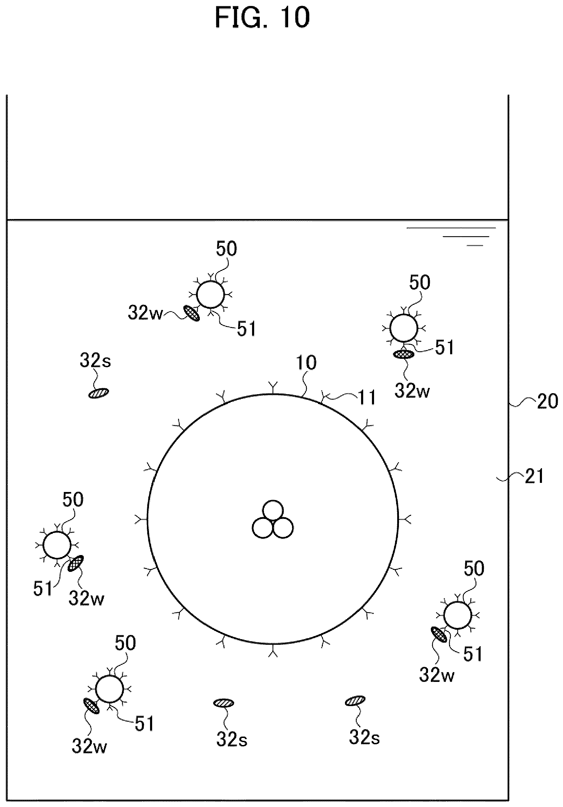

As illustrated in FIG. 10, in step S15, the operator performs the process of detaching the exosomes 32 from the magnetic microbead 10. Thereafter, the double-positive exosomes 32w having the antibody nanobeads 50 bound thereto and the single-positive exosome 32s not having the antibody nanobeads 50 bound thereto are detached from the magnetic microbead 10.

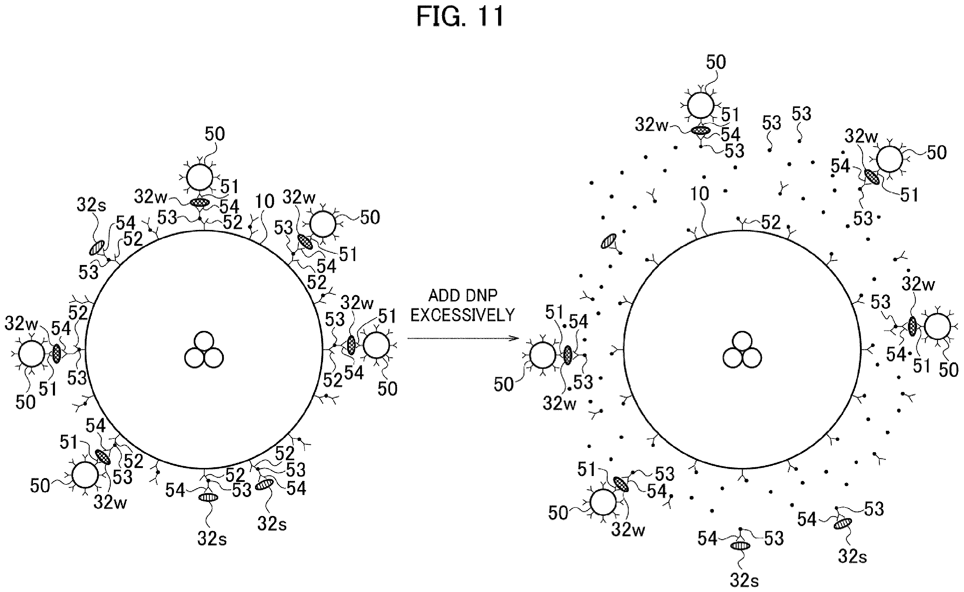

One of the following methods can be employed as the process for detaching the exosomes 32 from the magnetic microbead 10 in step S15. As a first method, a hapten shown in FIG. 11 is used. A hapten is a substance that binds to an antibody but, because of its low molecular weight, does not show any activity (immunogenicity) of inducing antibody production. A hapten becomes an immunogenic complete antigen when bound to an appropriate protein.

As illustrated in FIG. 11, the anti-DNP antibodies 52 are fixed to the magnetic nanobead 10 in place of the antibodies 51. DNP is 2,4-dinitrophenol, which is an example of a hapten. The double-positive exosome 32w or the single-positive exosome 32s is bound to the anti-DNP antibody 52 via an antibody 54 (DNP antibody 54) to which the DNP53 is bound. The DNP antibody 54 is an antibody that binds to CD147.

In this state, the binding of the anti-DNP antibody 52 to the DNP antibody 54 is an equilibrium reaction. When the DNP 53 (hapten) is excessively added to the buffer fluid 21 (third buffer fluid) containing the exosomes 32w sandwiched between the magnetic microbead 10 and the antibody nanobeads 50, the DNP antibody 54 bound to the anti-DNP antibody 52 is replaced by the DNP 53, and the magnetic microbead 10 is detached from the double-positive exosomes 32w bound to the antibody nanobeads 50, and the single-positive exosomes 32s.

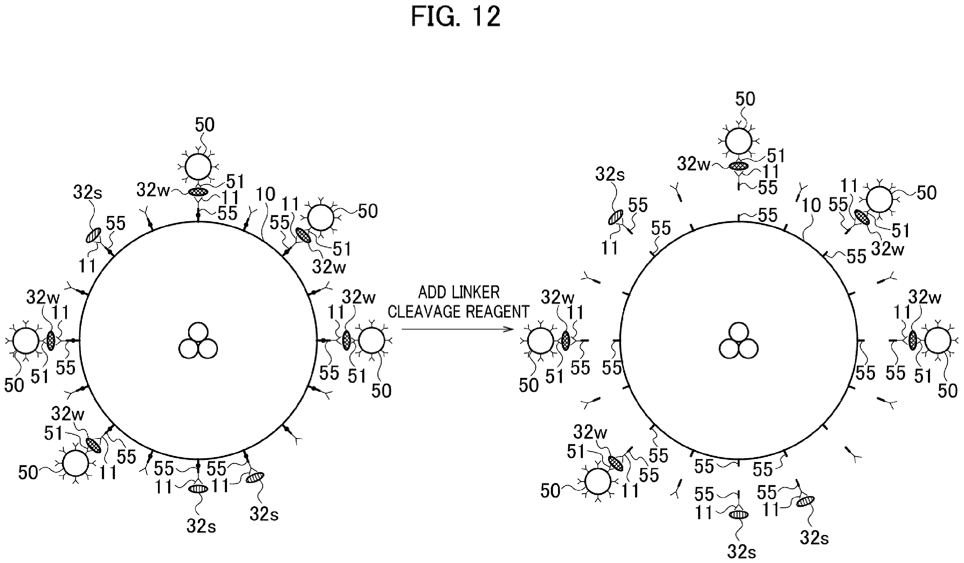

A second method of separating the exosomes 32 from the magnetic microbead 10 is to use a cleavable linker 55 illustrated in FIG. 12. The antibody 11 is fixed to the magnetic microbead 10 via the linkers 55. In a case where the second method is employed, the antibody 11 is fixed via the linker 55 to the magnetic microbead 10 illustrated in FIG. 8, and the antibody 11 is bound to the exosome 32.

When a linker cleavage reagent is added to the buffer fluid 21 (third buffer fluid) in the reaction container 20, the linker 55 is cleaved to detach the antibody 11 from the magnetic microbead 10.

As a third method of separating the exosome 32 from the magnetic microbead 10, a whole antibody may be used for the magnetic microbead 10 and a Fab antibody may be used for the antibody nanobead 50. Degradation and digestion of the whole antibody using enzymes allows the exosome 32 to be separated from the magnetic microbead 10.

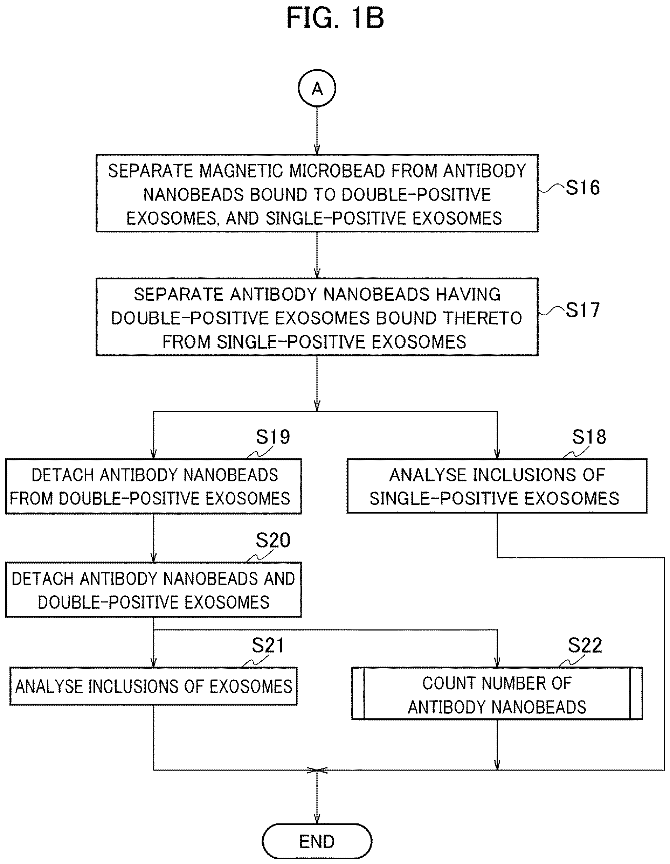

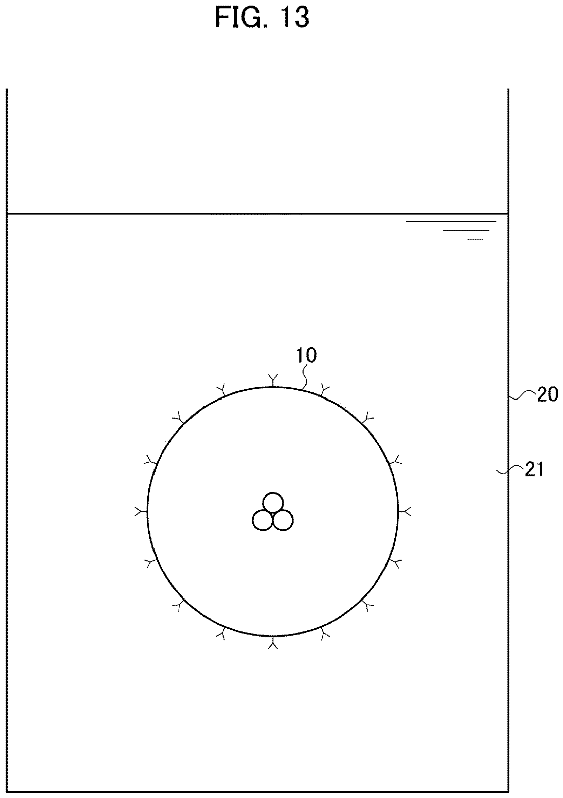

In FIG. 1B, the operator separates the magnetic microbead 10 from the antibody nanobead 50 bound to the double-positive exosome 32w, and the single-positive exosome 32s in step S16. It is possible to collect the magnetic microbead 10 illustrated in FIG. 13 by washing the separated magnetic microbead 10. In FIG. 13, the buffer liquid 21 containing only the magnetic microbead 10 is injected into the reaction container 20.

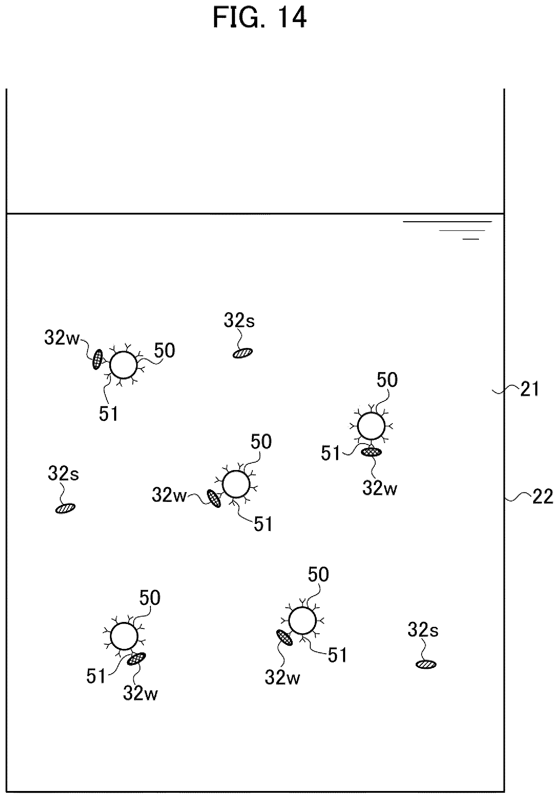

It is possible to collect the antibody nanobeads 50 bound to the exosomes 32w, and the exosomes 32s illustrated in FIG. 14 by washing the separated antibody nanobeads 50 bound to the exosomes 32w, and the exosomes 32s which have been separated. In FIG. 14, the buffer fluid 21 containing the antibody nanobeads 50 bound to the exosomes 32w, and the exosomes 32s is injected into the container 22.

Separation of the antibody nanobeads 50 bound to the exosomes 32w, and the exosomes 32s from the magnetic microbeads 10 in step S16 can be performed in the same manner as in FIG. 9. However, regarding the separation performed in step S16, it is necessary to collect the antibody nanobeads 50 bound to the exosomes 32w, and the exosomes 32s without removing the supernatant. Here, the antibody nanobeads 50 bound to the exosomes 32w, and the exosomes 32s are contained in the supernatant.

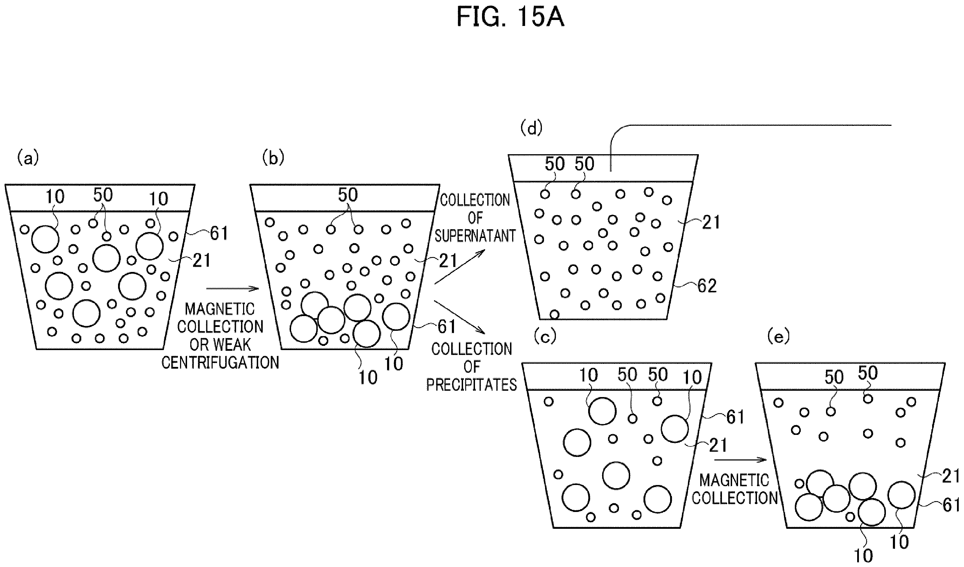

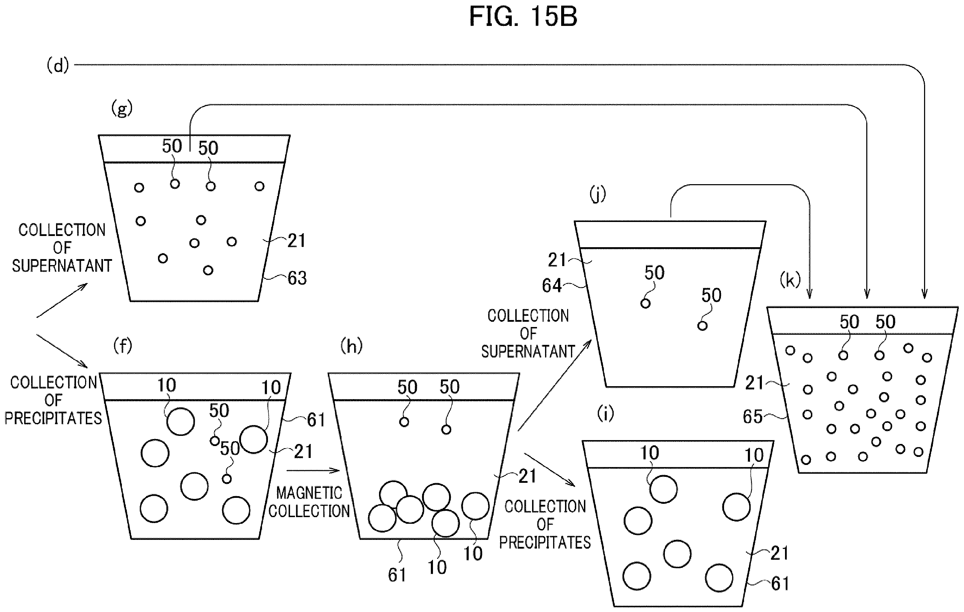

Specifically, as illustrated in FIGS. 15A and 15B, the magnetic microbeads 10 and the antibody nanobeads 50 bound to the exosomes 32w can be separated. The exosomes 32s are not illustrated in FIGS. 15A and 15B.

In FIG. 15A, (a) illustrates a state where a container 61 is injected with the buffer fluid 21 which contains the magnetic microbeads 10 and the antibody nanobeads 50 separated from each other. Part (a) of FIG. 15A corresponds to the state of FIG. 10. Magnetic collection in which a magnet is placed at the bottom of the container 61 or weak centrifugation can mostly separate the magnetic microbeads 10 from the antibody nanobeads 50, as illustrated in (b) of FIG. 15A.

When the precipitates, which are mainly the magnetic microbeads 10 in (b) of FIG. 15A, are collected and the buffer fluid 21 is added, the antibody nanobeads 50 can be greatly reduced as illustrated in (c) of FIG. 15A. As illustrated in (d) of FIG. 15A, the supernatant is collected into the container 62 to obtain the buffer fluid 21 containing a large number of the antibody nanobeads 50. When magnetic collection is performed in the state illustrated in (c) of FIG. 15A, (e) of FIG. 15A is achieved.

When the precipitates, which are mainly the magnetic microbeads 10 in (e) of FIG. 15A, are collected and the buffer fluid 21 is added, the antibody nanobeads 50 can be further reduced as illustrated in (f) of FIG. 15B. As illustrated in (g) of FIG. 15B, the supernatant is collected into the container 63 to obtain the buffer fluid 21 containing the antibody nanobeads 50. When magnetic collection is performed in the state illustrated in (f) of FIG. 15B, (h) of FIG. 15B is achieved.

When the precipitates, which are only the magnetic microbeads 10 in (h) of FIG. 15B, are collected and the buffer fluid 21 is added, the buffer fluid 21 containing only the magnetic microbeads 10 and not containing the antibody nanobeads 50 can be obtained as illustrated in (i) of FIG. 15B. Part (i) of FIG. 15B corresponds to the state of FIG. 13. As illustrated in (j) of FIG 15B, the supernatant is collected into the container 64 to obtain the buffer fluid 21 containing a small number of the antibody nanobeads 50.

As illustrated in (k) of FIG. 15B, when the antibody nanobeads 50 in all of the containers 62 to 64 are combined, all the antibody nanobeads 50 which were bound to the magnetic microbeads 10 and are bound to the exosomes 32w can be collected. At this time, the exosomes 32s can also be collected. Part (k) of FIG. 15B corresponds to the state in FIG. 14. The number of times the precipitates are collected and the supernatant is removed is not limited to the number of times illustrated in FIGS. 15A and 15B.

In FIG 1B, the operator separates the antibody nanobeads 50 bound to the exosomes 32w from the exosomes 32s in step S17. The antibody nanobeads 50 bound to the exosomes 32w, and the exosomes 32s can be separated and collected individually by the same method as in FIGS. 15A and 15B.

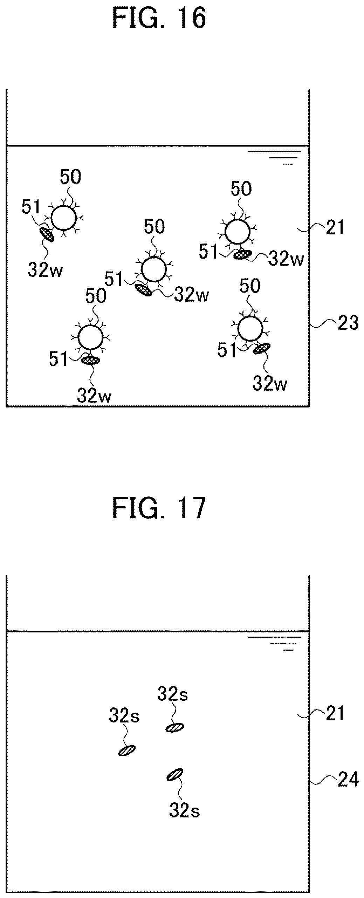

It is possible to collect the antibody nanobeads 50 bound to the exosomes 32w illustrated in FIG. 16 by washing the separated antibody nanobeads 50 bound to the exosomes 32w. In FIG. 16, the buffer fluid 21 containing only the antibody nanobeads 50 bound to exosomes 32w is injected into the container 23. It is possible to collect the exosomes 32s illustrated in FIG. 17 by washing the separated exosomes 32s. In FIG. 17, the buffer fluid 21 containing only the exosomes 32s is injected into the container 24.

The operator dissolves the exosomes 32s and analyzes the inclusions of the exosomes 32s in step S18. When a surfactant is added to the buffer fluid 21 containing the exosomes 32s, the lipid double membranes 320 of the exosomes 32s are dissolved and broken. In one or more embodiments, since the primary objective is to analyze the double-positive exosomes 32w, this step S18 may be omitted.

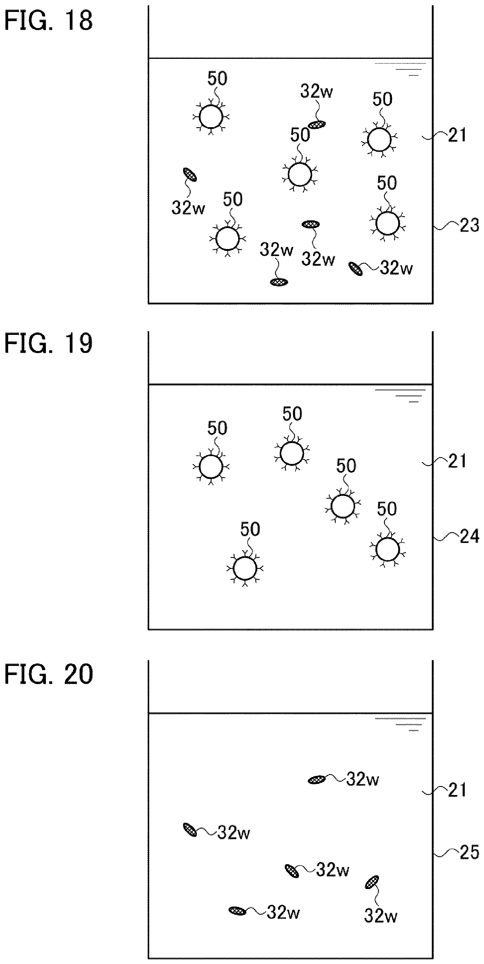

The operator performs the process of detaching the antibody nanobeads 50 from the exosomes 32w in step S19. As an example, the addition of a surfactant to the buffer fluid 21 illustrated in FIG. 16 can separate the antibody nanobeads 50 from the exosomes 32w, as illustrated in FIG. 18. For example, the surfactant is a Triton X-100 solution, which is a 2% nonionic surfactant. Since the lipid double membranes 320 of the exosomes 32w are dissolved by this surfactant, the exosomes 32w are broken to separate the antibody nanobeads 50 from the exosomes 32w, and the inclusions of the exosomes 32w are released into the buffer fluid 21. In FIG. 18, as the lipid double membranes 320 of the exosomes 32w are broken, the exosomes 32w are actually broken into a plurality of inclusions.

In step S20, the operator separates and individually collects the antibody nanobeads 50 and the inclusions of the exosomes 32w. The antibody nanobeads 50 and the inclusions of the exosomes 32w can be separated by the same method as in FIGS. 15A and 15B.

It is possible to collect the antibody nanobeads 50 illustrated in FIG. 19 by washing the separated antibody nanobeads 50. In FIG. 19, the buffer fluid 21 containing only the antibody nanobeads 50 is injected into the container 24. It is possible to collect the inclusions of the exosomes 32w illustrated in FIG. 20 by washing the inclusions of the separated exosomes 32w. In FIG. 20, the respective exosomes 32w are actually broken into a plurality of inclusions. In FIG. 20, the buffer fluid 21 containing only the inclusions of the exosomes 32w is injected into the container 25.

In step S21, the operator removes the inclusions of the exosomes 32w into the container 25, and analyzes the inclusions. At this time, a DNA or a protein in the inclusions can be analyzed by an analytical method such as RT-PCR (reverse transcription-polymerase chain reaction) analysis or LC-MS (liquid chromatography-mass spectrometry) analysis. In step S22, the operator counts the number of antibody nanobeads 50. The operator may perform steps S21 and S22 in an optional order, or these steps may be performed simultaneously by a plurality of operators. The order of steps S18, S21 and S22 is also optional. When the operator has performed steps S21 and S22 (or steps S18, S21 and S22), the process of the biological sample analysis is completed.

Since the antibody nanobeads 50 that are counted in step S22 are bound to the double-positive exosomes 32w, the number of the exosomes 32w in which both CD147 and HER2 are present is counted in step S22. The exosomes 32 in which the inclusions are analyzed in step S21 are only the double-positive exosomes 32w from which the single-positive exosomes 32s are removed, that is, the exosomes 32w that are counted in step S22.

Thus, the double-positive exosomes 32w in which the inclusions are analyzed are the same as the double-positive exosomes 32w that are counted. In one analyte 31 illustrated in FIG. 4, the inclusions of the double-positive exosomes 32w in which both CD147 and HER2 are present can be analyzed and the number of exosomes 32w can be counted. Here, each of CD147 and HER2, which are membrane proteins associated with specific diseases, is present in the exosomes 32w.

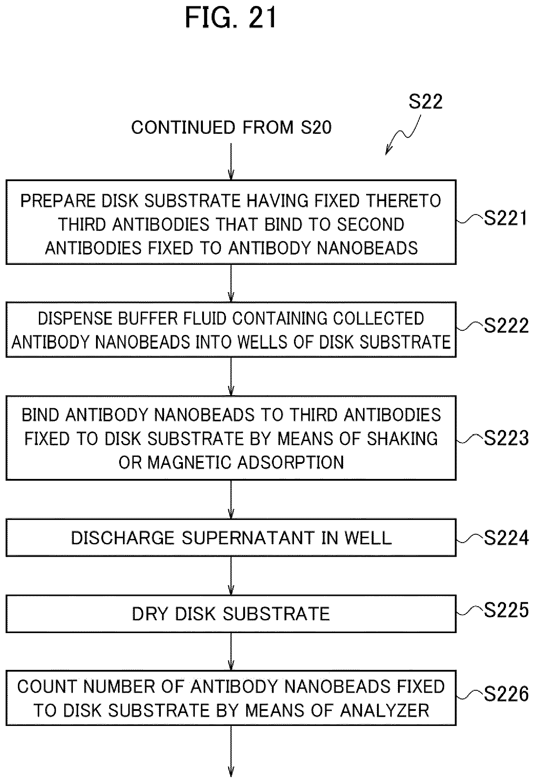

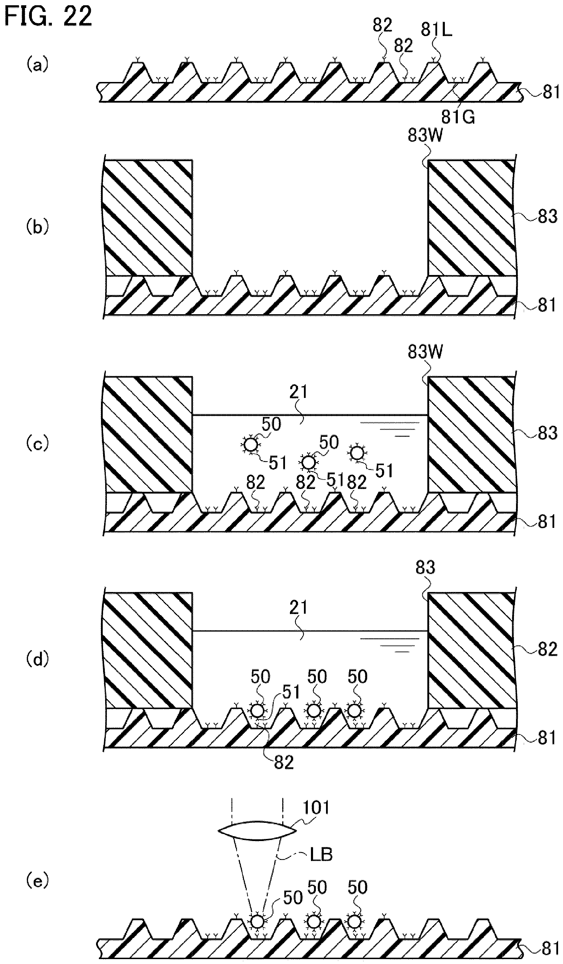

Referring to FIGS. 21 to 23, a description will be given regarding a specific method of counting the number of antibody nanobeads 50 in step S22 of FIG. 1B. In FIG. 21, the operator, in step S221, prepares a disk substrate 81 having antibodies 82 (third antibodies) fixed thereto, as illustrated in (a) of FIG. 22. The antibodies 82 bind to the antibodies 51 fixed to the antibody nanobeads 50. The antibody 82 is a goat anti mouse IgG antibody, for example.

The disk substrate 81 is preferably disk-shaped, and recesses 81G and projections 81L are alternately arranged in the radial direction. The recesses 81G and the projections 81L are formed in a spiral or a concentric shape.

As illustrated in (b) of FIG. 22, the operator mounts a cartridge 83 having a plurality of, for example, cylindrical through-holes formed therein on the disk substrate 81. Thereafter, a well 83W is formed by the disk substrate 81 and the cartridge 83. As illustrated in (c) of FIG. 22, the operator dispenses the buffer fluid 21 containing the collected antibody nanobeads 50 into each well 83W in step S222. It should be noted that the cartridge 83 may have the configuration disclosed in Patent Literature 2.

As illustrated in (d) of FIG. 22, the operator binds the antibodies 51 of the antibody nanobeads 50 to the antibodies 82 fixed to the disk substrate 81 by means of shaking or magnetic adsorption in step S223. When a magnet is placed on the rear surface of the disk substrate 81 using the antibody nanobeads 50 as magnetic nanobeads, the antibody nanobeads 50 are attracted to the surface of the disk substrate 81. Thus, the antibodies 51 of the antibody nanobeads 50 are easily bound to the antibodies 82 fixed to the disk substrate 81.

The operator discharges the supernatant in the well 83W in step S224, and dries the disk substrate 81 in step S225. As illustrated in (e) of FIG. 22, in step S226, the operator mounts the dried disk substrate 81 to the analyzer, and counts the number of antibody nanobeads 50 fixed to the disk substrate 81 by means of the analyzer. The analyzer counts the number of antibody nanobeads 50 by irradiating the disk substrate 81 with a laser beam condensed by a condensing lens 101. It should be noted that the analyzer may have the configuration disclosed in Patent Literature 3.

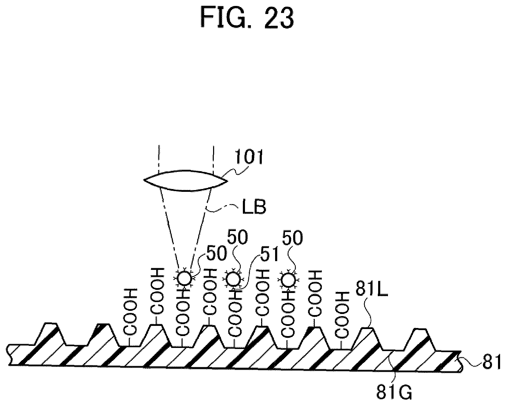

As illustrated in FIG. 23, instead of fixing the antibodies 82 to the disk substrate 81, the surface of the disk substrate 81 may be subjected to oxygen plasma treatment to form polar functional groups such as carboxyl groups (COOH). The antibodies 51 of the antibody nanobeads 50 are adsorbed to the polar functional groups.

As described above, in accordance with the biological sample analysis method according to one or more embodiments, using one biological sample (analyte 31), it is possible to analyze the inclusions of the double-positive exosomes 32w containing two disease-associated antigens (membrane proteins) and count the number of the exosomes 32w.

The present invention is not limited to one or more embodiments described above, and may be varied in various ways without departing from the spirit of the present invention. In one or more embodiments, the first bead is the magnetic microbead 10 and the second bead is the antibody nanobead 50, but the first bead and the second bead may be different in size so as to be separable from each other. When the first bead is a microbead and the second bead is a nanobead, they can be easily separated from each other. Thus, it is preferable that the first bead is a microbead and the second beads is a nanobead.

The first bead is preferably a magnetic bead, but may be a non-magnetic bead. When the first bead is a magnetic bead and the second bead is a non-magnetic bead, these beads can be separated from each other very easily. When the second bead is a magnetic bead, the second bead can be easily fixed to the disk substrate 81. The first bead may be a non-magnetic bead, and the second bead may be a magnetic bead. Further, both the first bead and the second bead may be a magnetic bead.

This present application claims priority under Japanese Patent Application No. 2020-024511 filed with the Japan Patent Office on February 17, 2020, the entire contents of all of which is incorporated herein by reference.

## Claims

1. A biological sample analysis method comprising:
injecting, into a reaction container, a first buffer fluid containing a plurality of a first bead having fixed to a surface thereof a first antibody that specifically binds to a first antigen associated with a first particular disease;
injecting, into the reaction container, a plurality of a second bead having fixed to a surface thereof a second antibody that specifically binds to a second antigen associated with a second particular disease, and a biological sample containing an exosome to be analyzed having the first antigen and the second antigen on a surface of the exosome, thereby generating a second buffer fluid containing the exosome to be analyzed having the first bead and the second bead bound thereto;
collecting the exosome to be analyzed having the first bead and the second bead bound thereto from the second buffer fluid;
separating the first bead from the collected exosome to be analyzed having the first bead and the second bead bound thereto, and collecting the exosome to be analyzed having the second bead bound thereto;
dissolving the collected exosome to be analyzed having the second bead bound thereto, to be separated into the second bead and an inclusion of the exosome to be analyzed, and collecting the second bead and the inclusion of the exosome to be analyzed; and
analyzing the inclusion of the collected exosome to be analyzed, and counting the number of collected second beads.

2. The biological sample analysis method according to claim 1, wherein
the first antibody is fixed to the first bead using a hapten, and
the first bead is detached by adding the hapten into a third buffer fluid containing the exosome to be analyzed having the first bead and the second bead bound thereto.

3. The biological sample analysis method according to claim 1, wherein
the first antibody is fixed to the first bead by a cleavable linker, and
the first bead is detached by adding the cleavable linker into a third buffer fluid containing the exosome to be analyzed having the first bead and the second bead bound thereto.

4. The biological sample analysis method according to any one of claims 1 to 3, wherein a surfactant is used for the dissolving.

5. The biological sample analysis method according to any one of claims 1 to 4, wherein the first bead is a magnetic bead.
